Europäisches Patentamt

European Patent Office

Office européen des brevets

(11) Veröffentlichungsnummer: **0 375 809**
**A1**

(12) **EUROPÄISCHE PATENTANMELDUNG**

(21) Anmeldenummer: **88203008.3**

(51) Int. Cl.⁵: **A61F 13/10**

(22) Anmeldetag: **27.12.88**

(43) Veröffentlichungstag der Anmeldung:
**04.07.90 Patentblatt 90/27**

(84) Benannte Vertragsstaaten:
**AT BE CH DE ES FR GB GR IT LI LU NL SE**

(71) Anmelder: **MACINTOSH N.V.**
**Mauritsweg 134**
**NL-6171 AK Stein(NL)**

(72) Erfinder: **Huntjens, Jozef Hubertus Gerardus**
**Hegge 108A**
**NL-6365 EG Schinnen(NL)**

(74) Vertreter: **Bleukx, Lucas Lodewijk Maria, Ir. et al**
**OCTROOIBUREAU DSM Postbus 9**
**NL-6160 MA Geleen(NL)**

(54) **Ellenbogenbandage.**

(57) Ein leicht abgewinkelter Schlauch (1) aus unterschiedlich elastischen Stoffteilen (4, 5) bildet eine Ellenbogenbandage. Bogenförmig ineinander übergehende Schenkel (2 und 3) des Schlauches (1) werden durch sich kreuzende Bänder (7), die sich über die volle Länge des Schlauches erstrecken in einer bestimmten Winkelstellung fixiert. Bandageninnenseitig ist mindestens ein in verschiedenen Position anbringbares Preßpolste (18) vorgesehen. Mit Hilfe der Bandage läßt sich sowohl ein Ellenbogengelenk in einem bestimmten Beugewinkel fixieren als auch eine dem Gelenk benachbarte Muskelpartie mit erhöhtem Druck gegen Knochenteile pressen und dadurch insbesondere eine Epikondylitis vermeiden oder therapieren.

Fig. 2

## Ellenbogenbandage

Die Erfindung betrifft eine Ellenbogenbandage in Form eines leicht abgewinkelten Schlauches. Dieser besteht aus einem elastischen Stoff und umfaßt Mittel sowohl zur Gelenkfixierung als auch zur erhöhten Kompression mindestens eines Muskelstranges der Flexoren- und/oder Extensorengruppe.

Es sind zahlreiche Ausführungsformen von Bandagen bekannt, die sich auch als Ellenbogenbandagen verwenden lassen. Eine Zielsetzung dieser Bandagen besteht darin, zur Vorbeugung einer Verletzung oder zur Heilung eines bereits verletzten Gelenkbereichs das Gelenk zu fixieren und es dadurch von Kraftbeanspruchungen in wechselnden Positionen zu bewahren. Eine weitere Zielsetzung ist es, zur Vorbeugung einer partiellen Trennung zwischen Muskelsträngen und Knochen oder zur Heilung eines derartigen bereits eingetretenen, sich sehr schmerzhaft auswirkenden Schadens auf die Muskelstränge einen Anpreßdruck in Knochenrichtung auszuüben.

Die bekannten Bandagen erfüllen die verschiedenen Anforderungen in sehr unterschiedlicher Weise und sind häufig weniger geeignet, insbesondere eine durch Überanstrengungen verursachte Epikondylitis am Schlagarm von Tennisspielern zu vermeiden bzw. zu therapieren.

Aus der EP 0 027 172 ist eine elastische Bandage in Schlauchform bekannt, die mit einer Kompressionseinlage versehen ist, welche in angelegtem Zustand der Bandage Knochenvorsprüngen benachbarte Gelenkweichteile beaufschlagen soll. Diese Bandage ist jedoch im übrigen so ausgebildet, daß die Gelenkfunktion ausdrücklich nicht beeinträchtigt wird, d. h. eine Gelenkfixierung damit nicht erfolgen kann und im speziellen Therapiefall auch nicht erfolgen soll.

Aus der US-PS 3 934 583 ist eine Bandage aus einem ebenen Stoffstück bekannt, das sich schlauchförmig beispielsweise um einen Ellenbogenbereich anordnen läßt und sich im wesentlichen auf die Aufgabe beschränkt, einen leicht abgewinkelten Arm in dieser Position zu halten bzw. Relativbewegungen zwischen dem Unter- und Oberarm einen gewissen Widerstand entgegenzusetzen. Diese Wirkungweise der Manschette wird durch sich kreuzende Bänder unterstützt, die sich diagonal über die Oberseite des im wesentlichen rechteckförmigen Stoffstückes erstrecken. Soweit mit dieser Manschette im angelegten Zustand Muskeln gegen Knochen anpreßbar sind, erfolgt dies über die gesamte Erstreckung der Bandage gleichförmig, was naturgemäß nicht nur eine Einschnürung durch die Bandage begünstigt, sondern auch die Möglichkeit ausschließt, spezifisch gefährdete oder bereits geschädigte Muskelpartien mit einem intensiveren Druck gegen Knochenteile zu pressen.

Der Erfindung liegt die Aufgabe zugrunde, eine Bandage der eingangs beschriebenen Art so auszubilden, daß sich damit sowohl ein Ellenbogengelenk starr in einer Position fixieren läßt, bei der Ober- und Unterarm einen Winkel kleiner als 180° einschließen, als auch dem Ellenbogenbereich benachbarte Muskelstränge der Flexoren-und Extensorengruppe vorzugsweise gegen Knochenteile wie Ulna und Radius partiell mit einem erhöhten Druck anpressen lassen, ohne daß dabei andere von der Bandage mit eingeschlossene Muskelstränge gleichfalls so komprimiert werden müssen, daß insgesamt eine Einschnürung zu befürchten ist. Insbesondere soll die Bandage so ausgebildet sein, daß sich mit ihr die durch Überanstrengungen verursachte Epikondylitis am Schlagarm von Tennisspielern vermeiden bzw. heilen läßt.

Zur Lösung dieser Aufgabe wird von einer Ellenbogenbandage der im Oberbegriff des Anspruchs 1 genannten gattungsgemäßen Art ausgegangen, welche erfindungsgemäß die im kennzeichnenden Teil desselben angegebenen Merkmale aufweist.

Durch die erfindungsgemäße Kombination der sich im Knickbereich kreuzenden Bänder, durch deren Zugfestigkeit die abgewinkelten Schlauchstücke in einer bestimmten Winkelposition fixierbar sind, und der Anordnung mindestens eines Preßpolsters auf der Innenseite des Schlauches, das einem Muskelstrang der Flexoren- oder Extensorengruppe gegenüberliegt, läßt sich einerseits das Ellenbogengelenk völlig entlasten und andererseits eine Muskelablösung verhindern oder aber eine bereits verursachte höchst wirksam therapieren. Die Gelenkfixierung und die gezielt lokalisierte Muskelkompression läßt den Schlagarm eines Tennisspielers nicht nur weiterhin voll funktionsfähig, sondern ermöglicht ihm dauerhaft eine optimale Schlagkraft zu übertragen, ohne dabei die typische Epikondylitis befürchten zu müssen oder deren Heilung zu beeinträchtigen. Ein weiterer Vorteil der erfindungsgemäßen Bandage besteht darin, daß die sich nur lokalisiert auswirkende erhöhte Kompression Einschnürungen nicht erforderlich macht und somit das Tragen der Bandage auch über längere Zeiträume nicht als belästigend empfunden wird.

Nach einer Ausgestaltung der Erfindung sind die Bänder jeweils mit ihrem einen Ende an gegenüberliegenden Bereichen eines Schlauchteils unlösbar und mit ihrem anderen Ende an gegenüberliegenden Bereichen eines anderen Schlauchteils lösbar befestigt.

Durch diese Ausgestaltung läßt sich der Abwinklungsgrad des Schlauches individuell variieren, wobei vorzugsweise die Bänder in ihrer Länge so bemessen sind, daß sich die lösbare Befestigung nur bewerkstelligen läßt, wenn ein bestimmter Abwinklungsgrad nicht unterschritten wird.

Nach einer weiteren Ausgestaltung der Erfindung ist das Preßpolster lösbar an verschiedenen Bereichen der Innenseite des Schlauches positionierbar.

Durch diese Ausgestaltung läßt sich die durch das Preßpolster hervorrufbare Kompression je nach den individuellen Bedürfnissen im Einzelfall gezielt zur Wirkung bringen, wobei erforderlichenfalls auch mehrere Preßpolster anbringbar sind, um zeitgleich verschiedene Muskelpartien mit erhöhter Kraft anzupressen.

Um den mit einem Preßpolster ausgeübten Druck individuell verstärken zu können, sieht eine weitere Ausgestaltung der Erfindung vor, daß auf der Außenseite des Schlauches ein das Polster überlagerndes, elastisch dehnbares, den Schlauch mindestens einmal umschlingendes Band angeordnet ist, das mit seinem einen Ende unlösbar am Schlauch und mit seinem anderen Ende lösbar am Band selbst befestigt ist.

Durch das die Manschette umschlingende Band läßt sich zudem eine unbeabsichtigte Verlagerung der Manschette verhindern, die selbst zu diesem Zweck den Ellenbogenbereich nicht allzu straff umspannen muß.

Überlagert nach einer weiteren Ausgestaltung der Erfindung das den Schlauch umschließende Band die lösbaren Enden der sich kreuzenden Bänder, so erfährt die Befestigung der Bandenden eine hochwirksame Unterstützung, so daß auch insoweit ein unbeabsichtigtes Lösen der Befestigung erschwert ist.

Nach einer weiteren Ausgestaltung der Erfindung schließen diametrale Längsstreifen des Schlauches jeweils eine mindestens in periferer Richtung biegesteife, sich über die Schlauchlänge erstreckende Einlage ein.

Im angelegten Zustand erfährt die Bandage durch die Einlagen eine zusätzliche Aussteifung, welche einer Veränderung des Schlauchknickwinkels einen hohen Widerstand entgegensetzt.

Die Steifigkeit der Bandage im angelegten Zustand läßt sich weiterhin dadurch optimieren, daß jeweils mit einem eine biegesteife Einlage nach außen abdeckenden Längsrandstreifen an einem Endbereich das unlösbare Ende und an einem anderen Endbereich das lösbare Ende der sich kreuzenden Bänder verbunden ist.

Durch diese Ausgestaltung ergibt sich eine fachwerkartige Aussteifung des Bereichs der Bandage, welcher der Ellenbeuge - also der Ellenbogeninnenseite - zugewandt ist, und stabilisiert damit die angelegte Bandage insgesamt in einer hervorragenden Weise.

Damit die biegesteifen Einlagen keine Druckstellen am Ober- oder Unterarm erzeugen, ist vorzugsweise jeweils einem eine biegesteife Einlage nach innen abdeckenden Längsrandstreifen eine Polsterung zugeordnet.

Zur Erleichterung des An- und Ablegens der Bandage weist nach einer besonders wesentlichen Ausgestaltung der Erfindung ein das Preßpolster aufnehmender, der Ulna und dem Radius zugeordneter Schenkel des Schlauches auf der der Ellenbeuge abgewandten Seite einen Längsschnitt auf, der durch sich überlappende und lösbar miteinander zu verbindende Mantelstücke des Schlauchs schließbar ist.

Die Bandage läßt sich somit leicht bei zunächst geöffnetem Schlitz des dem Unterarm zugeordneten Schlauchschenkels mit dem geschlossenen Schlauchschenkel auf den Oberarm in die vorgesehene Position schieben, worauf durch eine überlappende Anordnung der Mantelstücke des Schlauches der Schlitz geschlossen wird und die Bandage über ihre volle Länge den Ellenbogenbereich umschließt. Vorzugsweise sind die Mantelstücke am Schlitzende mit Aussparungen versehen, die bei im übrigen geschlossenen Schlitz eine Öffnung freilassen.

Schließlich sieht eine Ausgestaltung der Erfindung noch vor, daß alle lösbaren Verbindungen aus sogenannten Klettenverschlüssen bestehen, die sich durch vielfältige Vorteile auszeichnen und die sich vor allem ohne besonderes Geschick auch mit nur einer Hand schnell schließen und öffnen lassen.

In der Zeichnung ist ein Ausführungsbeispiel einer erfindungsgemäßen Bandage dargestellt und im folgenden näher beschrieben:

Es zeigen:

Fig. 1 eine Seitenansicht der Bandage im angelegten Zustand;

Fig. 2 die Bandage gemäß Fig. 1 in Blickrichtung auf die Ellenbeuge;

Fig. 3 ein abgebrochen dargestelltes Teilstück der Bandage in ausgebreitetem Zustand.

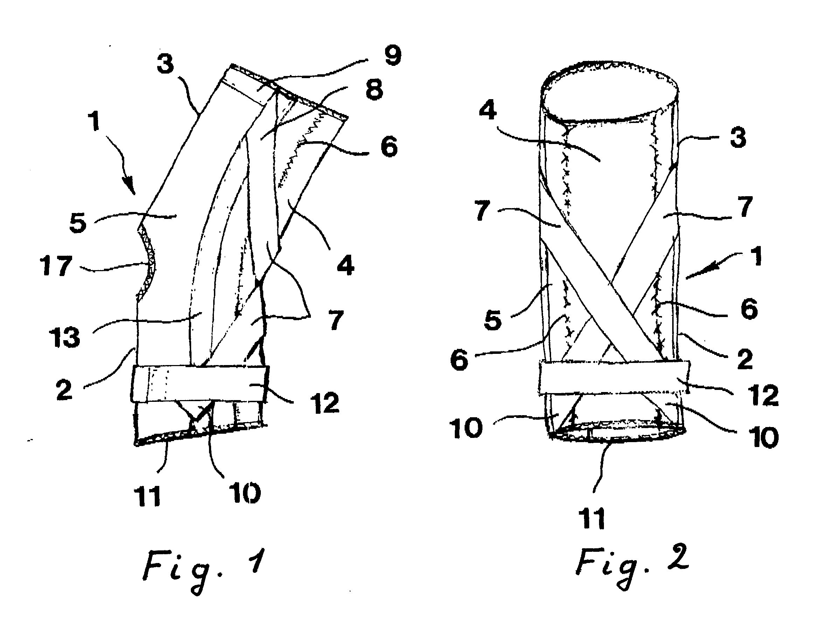

Wie Fig. 1 zeigt, hat die Bandage die Form eines leicht abgewinkelten Schlauchs 1, der aus einem elastischen Stoff gebildet ist. Der Schlauch 1 geht bogenförmig von einem unteren Schenkel 2, der einem Unterarm zuzuordnen ist, in einen oberen Schenkel 3, der einem Oberarm zuzuordnen ist, über und ist im wesentlichen aus einem besonders elastischen ellenbeugeseitig angeordneten Stoffteil 4 und einem weniger elastischen, der Ellenbeugeseite abgewandten Stoffteil 5 zusammengesetzt. Die schalenförmigen Stoffteile 4 und 5 sind durch Längsnähte 6 miteinander verbunden, wobei im Stoffteil eine nicht dargestellte atmungsaktive Einlage eingearbeitet ist.

Auf der Ellenbeugeseite sind zwei sich kreuzende Bänder 7 angeordnet, die sich über die Länge des Schlauches 1 erstrecken und ihren Kreuzungspunkt im Übergangsbereich zwischen den Schenkeln 2 und 3 haben. Die Bänder 7 sind jeweils mit einem Ende 8 im Bereich eines Schlauchendteils 9 unlösbar und mit einem anderen Ende 10 am gegenüberliegenden Bereich eines Schlauchendteils 11 mit Hilfe eines nicht dargestellten Klettenverschlusses lösbar befestigt.

Im Bereich der lösbar befestigten Enden 11 der Bänder 7 umschlingt ein weiteres Band 12 den unteren Schenkel 2 und greift mit einem unlösbar befestigten Ende an einem Längsstreifen 13 an, der sich im bogenförmigen Verlauf des Schlauches 1 über dessen gesamte Länge erstreckt und von dem ein weiteres Exemplar in gleicher Weise auf der gegenüberliegenden Seite spiegelbildlich angeordnet ist.

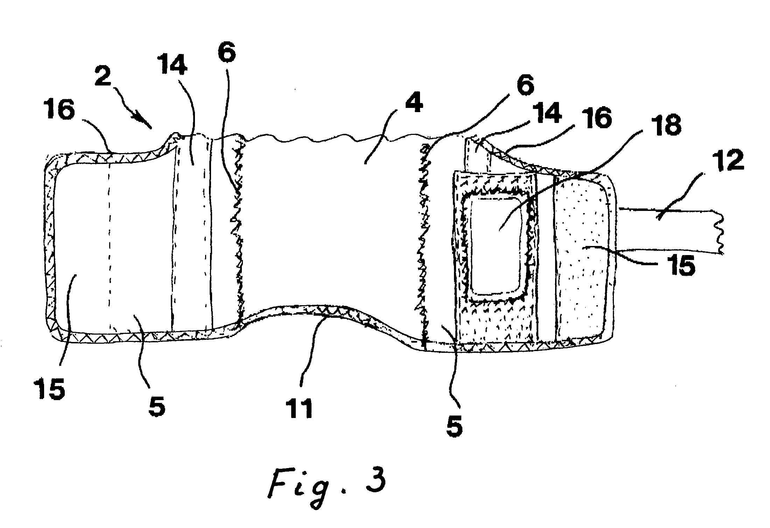

Diese Längsstreifen 13, an deren obere Ende auch die unlösbar befestigten Enden 8 der Bänder 7 angreifen, decken je eine biegesteife Einlage ab, die bandageninnenseitig mit einer in Fig. 3 erkennbaren Polsterung 14 versehen ist.

Wie Fig. 3 deutlich zeigt, ist der untere Schenkel 2 des Schlauches 1 axial aufgeschnitten und erhält die Schlauchform erst dadurch, daß Mantelstücke 15 überlappend miteinander verbunden werden. Der bogenförmige Verlauf einer oberen Begrenzungskante 16 der Mantelstücke 15 führt in angelegtem Zustand der Bandage dazu, daß eine in Fig. 1 erkennbare Öffnung 17 verbleibt.

Wesentlicher Bestandteil des unteren Schenkel 2 ist ein Preßpolster 18, das wiederum mit Hilfe eines Klettenverschlusses an der Innenseite einer der Mantelstücke 15 so angebracht ist, daß sich das Preßpolster 18 im angelegten Zustand der Bandage mit erhöhtem Druck gegen Muskelstränge der Flexoren- oder Extensorengruppe anpressen läßt. Dabei kann der Grad der Anpressung je nach der Spannkraft, mit der das elastische Band 12 um den unteren Schenkel 2 gebunden wird, variiert werden, da das Band 12 so angeordnet ist, daß es nicht nur die lösbaren Enden 10 der Bänder 7 sondern auch das Preßpolster 18 überlagert.

**Ansprüche**

1. Ellenbogenbandage in Form eines leicht abgewinkelten Schlauches aus einem elastischen Stoff sowie Mitteln zur Gelenkfixierung und zur erhöhten Kompression mindestens eines Muskelstranges der Flexoren- und/oder Extensorengruppe, gekennzeichnet, durch mindestens zwei auf der Außenseite des Schlauches (1) sich im Knickbereich kreuzend anbringbare und sich über die Gesamtlänge des Schlauches (1) erstreckende zugfeste Bänder (7) sowie mindestens ein auf der Innenseite des Schlauches (1) der Flexoren- oder Extensorengruppe gegenüberliegend angeordnetes Preßpolster (17).

2. Bandage nach Anspruch 1, dadurch gekennzeichnet, daß die Bänder (7) jeweils mit ihrem einen Ende (8) an gegenüberliegenden Bereichen eines Schlauchendteils (9) unlösbar und mit ihrem anderen Ende (10) an gegenüberliegenden Bereichen eines anderen Schlauchendteils (11) lösbar befestigt sind.

3. Bandage nach Anspruch 1, dadurch gekennzeichnet, daß das Preßpolster (18) lösbar an verschiedenen Bereichen der Innenseite des Schlauches (1) positionierbar ist.

4. Bandage nach mindestens einem der Ansprüche 1 bis 3, dadurch gekennzeichnet, daß auf der Außenseite des Schlauches (1) ein das Polster (18) überlagerndes, elastisch dehnbares, den Schlauch (1) mindestens einmal umschlingendes Band (12) angeordnet ist, das mit seinem einen Ende unlösbar am Schlauch (1) und mit seinem anderen Ende lösbar am Band (12) befestigt ist.

5. Bandage nach mindestens einem der Ansprüche 1 bis 4, dadurch gekennzeichnet, daß das den Schlauch (1) umschlingende Band (12) die lösbaren Enden (10) der sich kreuzenden Bänder (7) überlagert.

6. Bandage nach mindestens einem der Ansprüche 1 bis 5, dadurch gekennzeichnet, daß diametrale Längsstreifen (13) des Schlauches (1) jeweils eine mindestens in periferer Richtung biegesteife, sich über die Schlauchlänge erstreckende Einlage einschließen.

7. Bandage nach mindestens einem der Ansprüche 1 bis 6, dadurch gekennzeichnet, daß jeweils mit einem eine biegesteife Einlage nach außen abdeckenden Längsstreifen (13) an einem Endbereich das unlösbare Ende (8) und an einem anderen Endbereich das lösbare Ende (10) der sich kreuzenden Bänder (7) verbunden ist.

8. Bandage nach mindestens einem der Ansprüche 1 bis 7, dadurch gekennzeichnet, daß jeweils einem eine biegesteife Einlage nach innen abdeckenden Längsrandstreifen eine Polsterung (14) zugeordnet ist.

9. Bandage nach mindestens einem der Ansprüche 1 bis 8, dadurch gekennzeichnet, daß ein das Preßpolster (17) aufnehmender der Ulna und dem Radius zugeordneter Schenkel (2) des Schlauches (1) auf der der Ellenbeuge abgewandten Seite einen Längsschlitz aufweist, der durch sich überlappende und lösbar miteinander zu verbindende Mantelstücke (15) des Schlauchs (1) schließbar ist.

10. Bandage nach mindestens einem der Ansprüche 1 bis 9, dadurch gekennzeichnet, daß alle lösbaren Verbindungen aus sogenannten Kletten-

verschlüssen bestehen.

Fig. 1

Fig. 2

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | US-A-3 934 583 (HOLLINGSHEAD)<br>* Spalte 8, Zeilen 33-52; Figuren 11-13 * <br>--- | 1,10 | A 61 F 13/10 |
| Y | US-A-4 299 214 (SWEITZER)<br>* Spalte 3, Zeile 59 - Spalte 4, Zeile 43; Figuren * | 1,10 | |
| A | | 9 | |
| A | US-A-3 785 371 (LEWIS)<br>* Zusammenfassung; Figuren *<br>--- | 1 | |
| A | WO-A-8 604 811 (WEIHERMÜLLER)<br>* Seite 4, Zeile 1 - Seite 6, Zeile 27; Figur *<br>--- | 1,10 | |
| A | US-A-4 441 493 (NIRSCHL)<br>* Spalte 3, Zeile 27 - Spalte 6, Zeile 16: Figuren *<br>--- | 1,6,7,9,10 | |
| A | US-A-4 693 241 (TRZNADEL)<br>* Spalte 1, Zeile 49 - Spalte 2, Zeile 57; Figuren 1-7 *<br>--- | 1-5,10 | RECHERCHIERTE SACHGEBIETE (Int. Cl.5)<br><br>A 61 F |
| A | DE-U-8 620 630 (RÖDER)<br>--- | | |
| A | US-A-3 942 525 (DRAGAN)<br>--- | | |
| A | US-A-4 763 901 (RICHTER)<br>--- | | |
| A | FR-A-2 596 980 (BERTHEAS)<br>----- | | |

Der vorliegende Recherchenbericht wurde für alle Patentansprüche erstellt

| Recherchenort | Abschlußdatum der Recherche | Prüfer |
|---|---|---|
| DEN HAAG | 24-08-1989 | KLEIN C. |

EPO FORM 1503 03.82 (P0403)